# EUROPÄISCHE PATENTSCHRIFT

(11) **EP 3 294 222 B1**
(45) Veröffentlichungstag und Bekanntmachung des Hinweises auf die Patenterteilung: **16.12.2020**
(21) Anmeldenummer: 16731494.7
(22) Anmeldetag: 09.05.2016
(51) Int. Cl.: A61F 2/28, A61F 2/30

(54) **VORRICHTUNG ZUR ABDECKUNG UND/ODER REKONSTRUKTION EINER KNOCHENDEFEKTSTELLE; VERFAHREN ZUR HERSTELLUNG EINES AUFSATZES EINER ABDECKUNG FÜR EINE KNOCHENDEFEKSTELLE**
DEVICE FOR COVERING AND/OR RECONSTRUCTING A BONE DEFECT SITE, METHOD FOR PRODUCING A CAP FOR A COVER FOR A BONE DEFECT SITE
DISPOSITIF POUR LE RECOUVREMENT ET/OU LA RECONSTRUCTION D'UN DÉFAUT D'UN OS ET PROCÉDÉ DE FABRICATION D'UN COURONNEMENT D'UN DISPOSITIF DE RECOUVREMENT POUR UN DÉFAUT D'UN OS

(30) Priorität: 08.05.2015 DE 102015006154; 11.01.2016 DE 102016000235
(43) Veröffentlichungstag der Anmeldung: 21.03.2018
(73) Patentinhaber: Reoss GmbH, 70794 Filderstadt (DE)
(72) Erfinder: SEILER, Marcus, 70597 Stuttgart (DE)
(74) Vertreter: Patentanwälte Schuster, Müller & Partner mbB
(86) Internationale Anmeldenummer: PCT/DE2016/000207
(87) Internationale Veröffentlichungsnummer: WO 2016/180397

(56) Entgegenhaltungen:
- EP-A2- 2 737 871
- WO-A1-96/12446
- WO-A2-2006/051401
- DE-A1-102011 011 191
- US-B1- 7 172 422

## Beschreibung

### Stand der Technik

Die Erfindung geht aus von einer Vorrichtung zur Abdeckung und/oder Rekonstruktion einer Knochendefektstelle, nach der Gattung des Anspruchs 1, und einem Verfahren zur Herstellung eines Aufsatzes einer Abdeckvorrichtung für eine Knochendefektstelle, nach der Gattung des Anspruchs 20.

Knochendefektstellen in Form von Ausnehmungen oder Höhlungen im körpereigenen Knochengewebe werden in der Knochenchirurgie, beispielsweise bei der Rekonstruktion von Knochen in der orthopädischen, neurochirurgischen oder plastischen Chirurgie oder bei kieferchirurgischen Operationen, oftmals mit Knochenaufbaumaterial gefüllt. In der Regel besteht das Knochenaufbaumaterial aus einer Mischung aus synthetischen Knochenersatzmaterial (z.B. Hydroxylapatitgranulat) und körpereigenen Knochenpartikeln. Damit das Knochenaufbaumaterial im Wesentlichen ausschließlich von der Knochenseite her knöchern durchwachsen wird, wird die Ausnehmung, wie in der Patentschrift DE 43 02 708 C2 beschrieben, mit einer Abdeckmembran verschlossen. Befestigt wird die Abdeckmembran mit Befestigungsnägeln am körpereigenen, an die Knochendefektstelle angrenzenden gesunden Knochen, der durch die Befestigungsnägel beschädigt wird, wobei, da die Abdeckmembran aus einem flexiblen Material besteht, die Befestigung ein Höchstmaß an handwerklichem Geschick des Chirurgen erfordert.

Um diesen Nachteil einer fehlenden Stützfunktion der Abdeckmembran zu überwinden, wird in der Patentschrift US 48 16 339 eine Abdeckmembran beschrieben, die aus mehreren Schichten besteht, wobei diese Schichten nicht aus resorbierbaren Membranmaterial bestehen. Dabei ist es gegebenenfalls erforderlich, dass nach dem Ausheilen des Knochendefektes ein zweiter Eingriff notwendig ist, um körperfremdes Material zu entfernen.

In der Patentschrift DE 10 2005 039 382 B4 wird ein biodegradierbarer Hohlkörper, der insbesondere eine hohlzylindrische oder kegelzylindrische Form aufweist, vorgeschlagen. Der Hohlkörper weist in seinen Wandungen eine Mehrzahl von Öffnungen auf, durch die eine Aufnahme von Blut und damit der Aufbau von Eigenknochen möglich ist. Nachteilig ist hierbei, dass zum Einsetzen des Hohlkörpers eine zylindrische Bohrung mittels eines Bohrers in den vorhandenen Knochen eingebracht werden muss.

In der Offenlegungsschrift DE 10 2006 047 054 A1 wird ein Implantatlager vorgeschlagen, das sich durch eine hohe Passgenauigkeit und Stabilität auszeichnet, so dass der behandelnde Arzt es einfach handhaben und implantieren kann. Das aus Hydroxylapatit angefertigte Implantatlager, das zum Schutz der Schleimhaut vor mechanischen Einwirkungen und zum Schutz des Implantatlagers vor einwachsenden Gewebe von Seiten der Schleimhaut auf der der Schleimhaut zugewandten Seite eine dünne, insbesondere aus resorbierbaren Material bestehende, Membran aufweist, wird mit einem aufbauenden Fertigungsverfahren hergestellt, so dass die Materialbeschaffenheit eine "Gradientenstruktur" im Sinne einer insbesondere nach innen abnehmenden Dichte bildet. Dabei ist an der dem Knochen zugewandten Seite eine Bauweise mit einer insbesondere porösen Struktur und an der Außenseite des Implantatlagers, an der sich eine Struktur zur Halterung eines Zahnimplantats und/oder eines Zahnersatzes befindet, eine kompakte Bauweise vorgesehen.

Des Weiteren werden in den Offenlegungsschriften DE 198 30 992 A1, DE 10 2005 060 761 A1, DE 42 26 465 A1, WO 01/91818 A1, DE 10 2005 041 412 A1, DE 10 2006 047 054 A1, US 2011/0151400 A1, WO 00/59409 A1 und WO 2006/051401 A2 und der Patentschrift US 7,172,422 B1 Vorrichtungen für eine Knochendefektstelle beschrieben, wobei sämtliche dieser Lösungen den Nachteil aufweisen, dass sie den neben der Knochendefektstelle vorhandenen gesunden Knochen in Mitleidenschaft ziehen.

### Die Erfindung und ihre Vorteile

Die erfindungsgemäße Vorrichtung zur Abdeckung und/oder Rekonstruktion einer Knochendefektstelle, wobei mit dem Begriff "Knochendefektstelle" eine Stelle eines (kranken, deformierten, verletzten, durch Alterungsprozesse veränderten, durch Degeneration (z.B. nach Zahnextraktion, Tumor usw.) veränderten und/oder im Volumen veränderten) Knochens (z.B. Hüfte, Wirbelsäule, Kopf, Kiefer odgl.) eines Menschen oder eines Tieres bezeichnet wird, die von der Form und/oder dem Volumen eines gesunden Knochens abweicht, mit den Merkmalen des Anspruchs 1, und das erfindungsgemäße Verfahren zur Herstellung eines Aufsatzes einer Abdeckvorrichtung für eine Knochendefektstelle, mit den Merkmalen des Anspruchs 20, haben demgegenüber den Vorteil, dass die Vorrichtung zur Abdeckung und/oder Rekonstruktion einer Knochendefektstelle aus einem einen Rand aufweisenden Aufsatz (z.B. Formschale, starre Schale, Formkörper), der ein- oder mehrschichtig ausgestaltet sein kann, und mindestens einem innerhalb der Knochendefektstelle anordbaren Fixiermittel zur Fixierung des Aufsatzes an einem Knochen besteht, wobei an dem Rand des Aufsatzes, der sich durch eine formstabile (starre) Beschaffenheit auszeichnet und zumindest teilweise (am Rand) im Grenzbereich zwischen der Knochendefektstelle und dem angrenzenden gesunden Knochen mit dem Knochen in Berührung steht und eine dem Knochendefekt zugewandte Wandung (im Sinne einer Oberfläche bzw. Seite) des Aufsatzes oder eine dem Knochendefekt abgewandte Wandung (im Sinne einer Oberfläche bzw. Seite) des Aufsatzes der Form des innerhalb der Knochendefektstelle regenerierten Knochens, der durch seine Regeneration wieder die Form eines gesunden Knochens aufweist, entspricht, mindestens ein öffnungsloses Positioniermittel angeordnet ist. Durch das mindestens eine Positioniermittel ist eine Positionierung des Aufsatzes an einem an die Knochendefektstelle angrenzenden gesunden Knochen möglich, da das Positioniermittel eine dem gesunden Knochen abgewandte Wandung (im Sinne einer Oberfläche bzw. Seite) und eine dem gesunden Knochen und mit diesem zumindest teilweise korrespondierende zugewandte Wandung (im Sinne einer Oberfläche bzw. Seite) aufweist. Der Aufsatz ist ausschließlich im Bereich der Knochendefektstelle, die von dem Aufsatz vollständig oder zumindest teilweise abgedeckt wird, angeordnet und/oder fixiert, so dass er den an die Knochendefektstelle angrenzenden gesunden Knochen, an dem aufgrund seiner Gesundheit sowieso keine Regeneration des Knochens stattfindet, nicht in Mitleidenschaft zieht. Statt der nur teilweisen Abdeckung der Knochendefektstelle durch den Aufsatz ist der Aufsatz somit bevorzugt passgenau auf die Knochendefektstelle abgestimmt und endet bevorzugt bündig am gesunden Knochen. Hierdurch ist die Knochendefektstelle vollständig durch den nicht über die Knochendefektstelle hinausragenden Aufsatz abgedeckt.

Nach einer vorteilhaften Ausgestaltung der erfindungsgemäßen Vorrichtung bestehen der Aufsatz und/oder mindestens ein Fixiermittel und/oder mindestens ein Positioniermittel mindestens teilweise aus einem biokompatiblen Werkstoff.

Nach einer zusätzlichen vorteilhaften Ausgestaltung der erfindungsgemäßen Vorrichtung ist der Werkstoff organischer und/oder anorganischer Herkunft. Dies kann auch ein autogener, syngener, allogener, xenogener, synthetischer oder alloplastischer Werkstoff sein.

Nach einer zusätzlichen vorteilhaften Ausgestaltung der erfindungsgemäßen Vorrichtung bestehen der Aufsatz und/oder mindestens ein Fixiermittel und/oder mindestens ein Positioniermittel mindestens teilweise aus einem biodegradierbaren Werkstoff.

Nach einer zusätzlichen vorteilhaften Ausgestaltung der erfindungsgemäßen Vorrichtung können der Aufsatz und/oder mindestens ein Fixiermittel und/oder mindestens ein Positioniermittel mindestens teilweise aus einem resorbierbaren Werkstoff bestehen. Vorteilhafterweise kann die Resorptionszeit der starren Schale durch deren Resorptionsgradienten gesteuert werden und/oder kann die Resorptionszeit auch weniger als sechs Monate betragen, so dass zeitnah das Implantat eingesetzt werden kann. Bevorzugt werden resorbierbare Metalle oder Legierungen, insbesondere Magnesium oder Magnesiumlegierungen, eingesetzt. Die 3D-Modelle (z.B. der Aufsatz und/oder das Fixiermittel) werden bevorzugt im Laserschmelz-Verfahren (Laser-melting-Verfahren) unter Vakuum aufgebaut, wobei bevorzugt ein 3D-Drucker zum Einsatz kommt.

Nach einer zusätzlichen vorteilhaften Ausgestaltung der erfindungsgemäßen Vorrichtung bestehen der Aufsatz und/oder mindestens ein Fixiermittel und/oder mindestens ein Positioniermittel mindestens teilweise aus einem Polymer oder einer polymeren Verbindung.

Nach einer zusätzlichen vorteilhaften Ausgestaltung der erfindungsgemäßen Vorrichtung bestehen der Aufsatz und/oder mindestens ein Fixiermittel und/oder mindestens ein Positioniermittel mindestens teilweise aus Polylactid. Polylactide sind aus vielen chemisch aneinander gebundenen Milchsäuremoleküle aufgebaut und gehören zu den Polymeren. Der Vorteil von Polylactid-Kunststoffen, die auch Polymilchsäuren (PLA) genannt werden, ist, dass sie durch Wärmezufuhr verformbare Kunststoffe und biokompatibel sind.

Nach einer zusätzlichen vorteilhaften Ausgestaltung der erfindungsgemäßen Vorrichtung weist der Aufsatz und/oder mindestens ein Positioniermittel eine konstante oder eine variierende Wandstärke auf.

Nach einer diesbezüglichen vorteilhaften Ausgestaltung der erfindungsgemäßen Vorrichtung sollte die Wandstärke mindestens 0,2 mm betragen, bevorzugt 0,5 mm, jedoch zumindest so viel, so dass sich eine Formstabilität der Formschale bzw. eines Positioniermittels ergibt.

Nach einer zusätzlichen vorteilhaften Ausgestaltung der erfindungsgemäßen Vorrichtung ist das Fixiermittel ein Pin, eine Schraube, ein Nagel und/oder ein Knochenkleber. Um den gesunden Knochen zu schonen, wird das bzw. werden die Fixiermittel bevorzugt im Bereich der Knochendefektstelle angeordnet.

Nach einer zusätzlichen vorteilhaften Ausgestaltung der erfindungsgemäßen Vorrichtung weist der Aufsatz mindestens eine Fräsung (Bohrung für das Fixiermittel) auf.

Nach einer diesbezüglichen vorteilhaften Ausgestaltung der erfindungsgemäßen Vorrichtung korrespondiert die Fräsung mit dem Fixiermittel.

Nach einer zusätzlichen vorteilhaften Ausgestaltung der erfindungsgemäßen Vorrichtung weist die dem Knochendefekt zugewandte Wandung eine Oberflächenkonditionierung auf.

Nach einer diesbezüglichen vorteilhaften Ausgestaltung der erfindungsgemäßen Vorrichtung kann die Oberfläche eine Mikrostrukturierung, Poren, Osteoblastenlockstoffe, Mittel zur Förderung des Knochenwachstums und/oder BMP-haltiges Knochenersatzmittel aufweisen.

Nach einer zusätzlichen vorteilhaften Ausgestaltung der erfindungsgemäßen Vorrichtung weist der Aufsatz mindestens eine Öffnung auf. Dies bedeutet, dass der Aufsatz keine geschlossene Wandung aufweisen muss. Durch eine Vielzahl von Öffnungen kann der Aufsatz zumindest stellenweise eine Netzstruktur aufweisen, wobei die dem Knochendefekt abgewandte Wandung der Netzstruktur oder die dem Knochendefekt zugewandte Wandung der Netzstruktur der Form des regenerierten Knochens entspricht.

Nach einer zusätzlichen vorteilhaften Ausgestaltung der erfindungsgemäßen Vorrichtung weist der Aufsatz mindestens eine Sollbruchstelle auf. Die Sollbruchstelle birgt den Vorteil, dass, sofern der Aufsatz nach einer erfolgreichen Knochenregeneration entfernt werden soll, diese Entfernung minimal invasiv erfolgen kann, ohne "alles offen legen zu müssen", da der Aufsatz aufgrund der Sollbruchstelle in mindestens zwei Teile zerkleinert werden kann. Die Entfernung des Aufsatzes (z.B. nach einer Knochenregeneration) ist somit sehr leicht möglich. Des Weiteren kann die Sollbruchstelle dazu dienen, dass nicht benötigte Teile des Aufsatzes von dem Rest des Aufsatzes abgetrennt werden.

Nach einer zusätzlichen vorteilhaften Ausgestaltung der erfindungsgemäßen Vorrichtung weist der Aufsatz mindestens eine Befestigungsvorrichtung (z.B. eine Aussparung) für mindestens ein zu setzendes Implantat auf.

Nach einer diesbezüglichen vorteilhaften Ausgestaltung der erfindungsgemäßen Vorrichtung ist mindestens eine Befestigungsvorrichtung (z.B. eine Aussparung) durch einen Teil des Aufsatzes, der mittels mindestens einer Sollbruchstelle mit dem restlichen Teil des Aufsatzes verbunden ist, zumindest teilweise abgedeckt.

Nach einer diesbezüglichen vorteilhaften Ausgestaltung der erfindungsgemäßen Vorrichtung ist zwischen dem Aufsatz und einem Positioniermittel mindestens eine Sollbruchstelle angeordnet. Dadurch kann ein Positioniermittel, das auf dem gesunden Knochen aufliegt und somit ggfls. störend aufträgt, z.B. nach Fixierung des Aufsatzes oder nach der Regeneration des Knochens an der Knochendefektstelle, von dem eventuell verbleibenden Aufsatz entfernt werden.

Nach einer vorteilhaften Ausgestaltung des erfindungsgemäßen Verfahrens zur Herstellung eines Aufsatzes einer Abdeckvorrichtung für eine Knochendefektstelle, bei dem die rechnergestützte Formgebung (CAD) des Aufsatzes mit einer rechnergestützten Fertigung (CAM) zu CAD/CAM kombiniert wird, so dass ein am Computer entwickeltes Entwurfsmodell des Aufsatzes direkt elektronisch an die Fertigung übermittelt wird, bestehend aus folgenden Verfahrensschritten:
- Aufnahme eines Datensatzes, der die betroffene Knochendefektstelle in seiner Dreidimensionalität repräsentiert, mittels Tomografie oder ähnlichen bildgebenden Verfahren,
- Verwendung des Datensatzes zur Planung des Aufsatzes, der eine dem Knochendefekt abgewandte Wandung und eine dem Knochendefekt zugewandte Wandung aufweist, und mit mindestens einem Fixiermittel an einem Knochen fixierbar ist,
- Umsetzung der Planung des Aufsatzes in einen Planungsdatensatz und
- Zuführung des Planungsdatensatzes an ein computergesteuertes Fertigungsverfahren,
wobei der Aufsatz aus einem formstabilen Material gebildet wird und dessen dem Knochendefekt zugewandte Wandung (im Sinne einer Oberfläche bzw. Seite) oder dessen dem Knochendefekt abgewandte Wandung ((im Sinne einer Oberfläche bzw. Seite) der Form des regenerierten Knochens entspricht, und die Aufnahme des Datensatzes, der die betroffene Knochendefektstelle in seiner Dreidimensionalität repräsentiert, mittels Tomografie, Computertomografie, digitaler Volumentomografie, Sonografie odgl., erfolgt, wobei während und/oder nach der Fertigung des Aufsatzes an dem Rand des Aufsatzes mindestens ein Positioniermittel angeordnet wird, das zur Positionierung des Aufsatzes an einem an die Knochendefektstelle angrenzenden gesunden Knochen an dem Aufsatz dient und das eine dem gesunden Knochen abgewandte Wandung (im Sinne einer Oberfläche bzw. Seite) und eine dem gesunden Knochen und mit diesem zumindest teilweise korrespondierende zugewandte Wandung (im Sinne einer Oberfläche bzw. Seite) aufweist, repräsentiert die Aufnahme des ersten Datensatzes die betroffene Knochendefektstelle in ihrer Dreidimensionalität und/oder repräsentiert die Aufnahme des zweiten Datensatzes die Form des noch gesunden Knochens in seiner Dreidimensionalität.

Nach einer zusätzlichen vorteilhaften Ausgestaltung des erfindungsgemäßen Verfahrens zur Herstellung eines Aufsatzes einer Abdeckvorrichtung für eine Knochendefektstelle erfolgen die Aufnahme des ersten Datensatzes, der den Ist-Zustand repräsentiert, und/oder die Aufnahme des zweiten Datensatzes, der den Soll-Zustand repräsentiert, durch mindestens ein bildgebendes Verfahren.

Nach einer zusätzlichen vorteilhaften Ausgestaltung des erfindungsgemäßen Verfahrens zur Herstellung eines Aufsatzes einer Abdeckvorrichtung für eine Knochendefektstelle die Aufnahme des ersten Datensatzes und/oder die Aufnahme des zweiten Datensatzes mit mindestens einem Verfahren, welches eine dreidimensionale Darstellung eines Knochens ermöglicht. Insbesondere erfolgen die Aufnahme des ersten Datensatzes und/oder die Aufnahme des zweiten Datensatzes mittels Tomografie, Computertomografie, digitaler Volumentomografie, Sonografie odgl..

Nach einer zusätzlichen vorteilhaften Ausgestaltung des erfindungsgemäßen Verfahrens zur Herstellung eines Aufsatzes einer Abdeckvorrichtung für eine Knochendefektstelle erfolgt die Aufnahme des Datensatzes des gesunden Knochens, nachdem der gesunde Knochen ausgewachsen ist. Dadurch ist es möglich, dass ggfls. der Idealzustand (Soll-Zustand) des Knochens dokumentiert wird, so dass bekannt ist, wie ein event. später zu regenerierender Knochen auszusehen hat. Bei Menschen sollte die Aufnahme des Datensatzes des gesunden Knochens bevorzugt im Alter von 18 bis 25 Jahren erfolgen. Selbstverständlich ist es auch denkbar, dass das im ausgewachsenen Zustand der Knochen mehrere gesunde Knochen oder das gesamte Skelett des Menschen oder des Tieres aufgezeichnet, dokumentiert und/oder gespeichert wird. Denkbar wäre auch, dass bereits zum Zeitpunkt der Aufnahme des gesunden Knochens ein Aufsatz zumindest teilweise angefertigt wird.

Nach einer diesbezüglichen vorteilhaften Ausgestaltung des erfindungsgemäßen Verfahrens zur Herstellung eines Aufsatzes einer Abdeckvorrichtung für eine Knochendefektstelle wird der Datensatz des gesunden Knochens für seine spätere Verwendung auf einem Speichermedium gespeichert (konserviert).

Nach einer zusätzlichen vorteilhaften Ausgestaltung des erfindungsgemäßen Verfahrens zur Herstellung eines Aufsatzes einer Abdeckvorrichtung für eine Knochendefektstelle wird im Fertigungsverfahren der Aufsatz mittels Fräsung gebildet.

Nach einer zusätzlichen vorteilhaften Ausgestaltung des erfindungsgemäßen Verfahrens zur Herstellung eines Aufsatzes einer Abdeckvorrichtung für eine Knochendefektstelle wird während der Fertigung des Aufsatzes mindestens eine Befestigungsvorrichtung für mindestens ein zu setzendes Implantat an dem Aufsatz angeordnet. Die Befestigungsvorrichtung kann beispielsweise als Aussparung ausgestaltet sein.

Nach einer diesbezüglichen vorteilhaften Ausgestaltung des erfindungsgemäßen Verfahrens zur Herstellung eines Aufsatzes einer Abdeckvorrichtung für eine Knochendefektstelle wird mindestens eine Befestigungsvorrichtung (z.B. Aussparung) durch Entfernen eines Teils des Aufsatzes, der vor dem Entfernen mittels mindestens einer Sollbruchstelle mit dem restlichen Teil des Aufsatzes verbunden ist, freigelegt. Der Zeitpunkt der Freilegung der Befestigungsvorrichtung kann dabei vor oder nach der Anordnung der Abdeckvorrichtung an der Knochendefektstelle liegen.

Nach einer zusätzlichen vorteilhaften Ausgestaltung des erfindungsgemäßen Verfahrens zur Herstellung eines Aufsatzes einer Abdeckvorrichtung für eine Knochendefektstelle wird während der Fertigung des Aufsatzes mindestens eine Sollbruchstelle an dem Aufsatz angeordnet.

Nach einer zusätzlichen vorteilhaften Ausgestaltung des erfindungsgemäßen Verfahrens zur Herstellung eines Aufsatzes einer Abdeckvorrichtung für eine Knochendefektstelle wird zwischen dem Aufsatz und einem Positioniermittel mindestens eine Sollbruchstelle angeordnet.

Nach einer zusätzlichen vorteilhaften Ausgestaltung des erfindungsgemäßen Verfahrens zur Herstellung eines Aufsatzes einer Abdeckvorrichtung für eine Knochendefektstelle wird während der Fertigung des Aufsatzes ein Reinigungs- und/oder Sterilisationsprozess durchgeführt.

Nach einer zusätzlichen vorteilhaften Ausgestaltung des erfindungsgemäßen Verfahrens zur Herstellung eines Aufsatzes einer Abdeckvorrichtung für eine Knochendefektstelle ist der Aufsatz in einer Vorrichtung zur Abdeckung und/oder Rekonstruktion einer Knochendefektstelle gemäß den Ansprüchen 1 bis 19 einsetzbar. Dadurch kann eine Vorrichtung zur Abdeckung und/oder Rekonstruktion einer Knochendefektstelle geschaffen werden, dessen Aufsatz und/oder Fixiermittel beispielsweise aus einem künstlichen Werkstoff und/oder aus einem Werkstoff autogener, synergener, allogener oder xenogener Herkunft menschliche und/oder tierische Knochen bzw. die menschliche, tierische oder künstliche Matrix eine Form auf, durch die der zwischen dem Knochen und der gewünschten Form des regenerierten Knochens befindlicher Bereich vollständig oder nahezu vollständig ausgefüllt wird. Hierzu wird dem Spender z.B. ein Knochenblock entnommen, der anschließend gegebenenfalls mittels CAD/CAM modelliert wird.

Durch das erfindungsgemäße Verfahren kann eine erfindungsgemäße Vorrichtung zur Abdeckung und/oder Rekonstruktion einer Knochendefektstelle geschaffen werden, dessen Aufsatz und/oder Fixiermittel beispielsweise aus einem Werkstoff organischer und/oder anorganischer Herkunft stammt. Dies kann auch ein synthetischer Werkstoff und/oder ein Werkstoff autogener, synergener, allogener und/oder xenogener, alloplastischer, menschlicher und/oder tierischer Herkunft sein. Dabei kann auch die menschliche, tierische oder synthetische Matrix eine Form aufweisen, durch die der zwischen dem Knochen und der gewünschten Form des regenerierten Knochens befindlicher Bereich vollständig oder nahezu vollständig ausgefüllt wird. Hierzu wird dem Spender (Eigen- oder Fremdspender) z.B. ein Knochenblock entnommen, der anschließend gegebenenfalls mittels CAD/CAM modelliert wird.

Weitere Vorteile und vorteilhafte Ausgestaltungen der Erfindung sind der nachfolgenden Beschreibung, der Zeichnung und den Ansprüchen entnehmbar.

### Zeichnung

Ausführungsbeispiele des Gegenstandes der Erfindung sind in der Zeichnung dargestellt und werden im Folgenden näher erläutert. Es zeigen:
- Fig. 1: eine Darstellung einer erfindungsgemäßen Vorrichtung zur Abdeckung und/oder Rekonstruktion einer Knochendefektstelle,
- Fig. 2: eine Darstellung einer anders geformten erfindungsgemäßen Vorrichtung zur Abdeckung und/oder Rekonstruktion einer Knochendefektstelle,
- Fig. 3: eine Darstellung einer anders geformten erfindungsgemäßen Vorrichtung zur Abdeckung und/oder Rekonstruktion einer Knochendefektstelle,
- Fig. 4: eine Darstellung einer anders geformten erfindungsgemäßen Vorrichtung zur Abdeckung und/oder Rekonstruktion einer Knochendefektstelle,
- Fig. 5: einen Ausschnitt eines Aufsatzes,
- Fig. 6 bis 8: verschiedene Darstellungen eines Aufsatzes mit Positioniermitteln und
- Fig. 9: einen an einer Knochendefektstelle angeordneten Aufsatz.

### Beschreibung der Ausführungsbeispiele

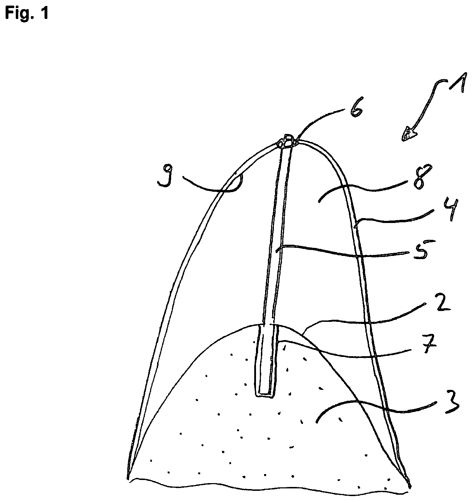

Fig. 1 zeigt eine Darstellung einer erfindungsgemäßen Vorrichtung 1 zur Abdeckung und/oder Rekonstruktion einer Knochendefektstelle 2 (Knochendefekt) eines Knochens, insbesondere eines Kieferknochens 3. Die Vorrichtung 1 besteht aus einem Aufsatz 4, der einschichtig ist, und einem Fixiermittel 5, das, um den an die Knochendefektstelle 2 angrenzenden gesunden Knochen nicht zu verletzen, in Fig. 1 als Pin dargestellt in der Knochendefektstelle 2 angeordnet ist. Selbstverständlich ist es auch denkbar, das mehrere Fixiermittel 5 zur Fixierung des Aufsatzes 4 eingesetzt werden, wobei diese ebenfalls in der Knochendefektstelle 2 angeordnet wären. Der Aufsatz 4 ist aus einem formstabilen Material, so dass er selbsttragend ist und keine zusätzliche Abstützung notwendig ist. Zur Fixierung des Aufsatz 4 (Formschale, starre Schale) wird das Fixiermittel 5 durch eine Bohrung 6 in den Aufsatz 4 geschoben und anschließend in die im Kieferknochen 3 angeordnete Bohrung 7 eingeführt. Die anschließende Fixierung des Aufsatzes 4 erfolgt bevorzugt über Ultraschall-Schweißen. Beim Ultraschall-Schweißen erzeugt bevorzugt ein Ultraschallgenerator eine genau definierte Frequenz, welche über eine Sonotrode gebündelt wird. Nach dem Aufsetzen des resorbierbaren Fixiermittels 5 (Pin) auf ein im Knochen vorgebohrtes Bohrloch (Bohrung 7) sorgt eine erzeugte Schwingung für eine Verflüssigung der Pinoberflächen an dessen Rändern, wodurch ein Eingleiten des Pins in das Bohrloch bewirkt wird. Durch die Änderung des Aggregatzustandes dringt der Pin auch in die knöchernen Hohlräume vor, die von einer gewöhnlichen Knochenschraube unerreichbar sind, so dass eine hohe initiale Festigkeit erzielt wird. Zudem verbindet sich der Pinkopf mit dem Aufsatz 4 und sorgt mit einem Verblockungsmechanismus für ein stabiles dreidimensionales Konstrukt. Beim Ultraschall-Schweißen wird somit das Fixiermittel 5 aufgeweicht, so dass es sich mit dem Kieferknochen 3 und dem Aufsatz 4 verbindet. Durch den fixierten Aufsatz 4 entsteht ein abgedichteter Innenraum 8 zwischen dem Kieferknochen 3 und dem Aufsatz 4, der durch die Regeneration des Knochens und/oder durch Einbringung eines Materials organischer und/oder anorganischer Herkunft, das auch ein autogener, syngener, allogener, xenogener, synthetischer und/oder alloplastischer Werkstoff sein kann, ausgefüllt wird, so dass der regenerierte Knochen oder das eingebrachte Material der Form der der Knochendefektstelle 2 zugewandten Wandung 9 (im Sinne einer Oberfläche bzw. Seite) des einschichtigen Aufsatzes 4 entspricht. Um den Regenerationsprozess des Kieferknochens 3 zu beschleunigen, kann die dem Knochendefekt zugewandte Wandung 9 des Aufsatzes 4 eine Oberflächenkonditionierung (z.B. eine Mikrostrukturierung, Poren, Osteoblastenlockstoffe, Mittel zur Förderung des Knochenwachstums und/oder BMP-haltiges Knochenersatzmittel) aufweisen.

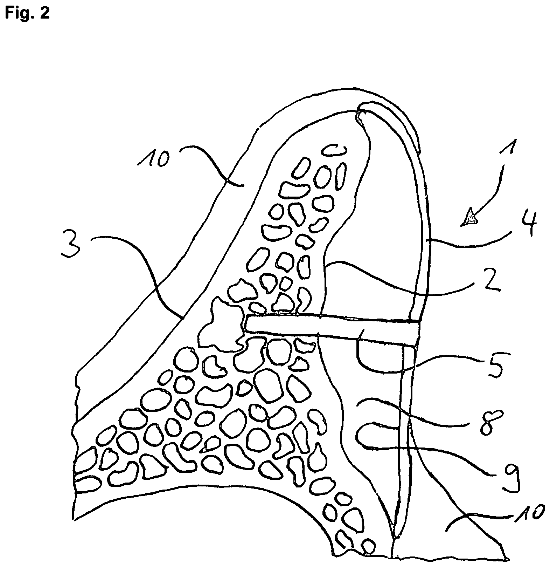

Fig. 2 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist zusätzlich das Zahnfleisch 10 angedeutet.

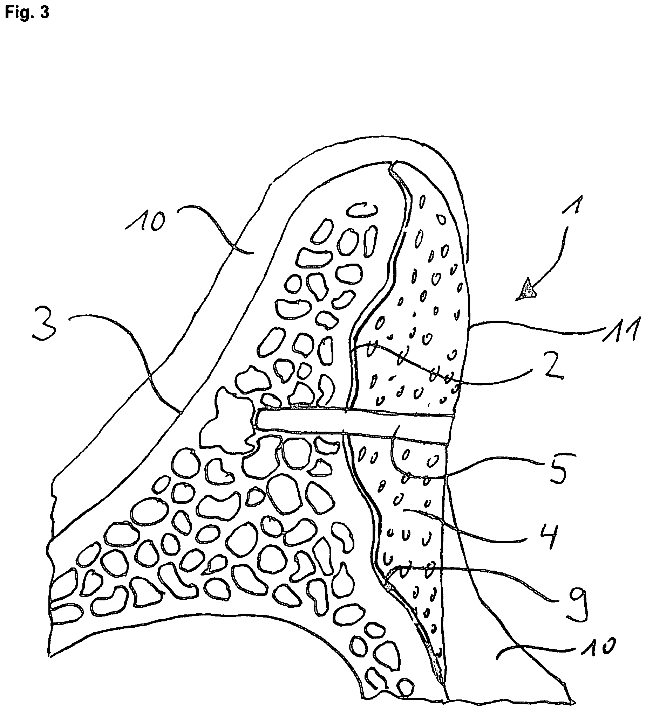

Fig. 3 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist der Aufsatz 4 als Formkörper z.B. aus menschlichen oder tierischen Knochen geformt und weist eine die dem Knochendefekt zugewandte Wandung 9 (im Sinne einer Oberfläche bzw. Seite), die an das Relief der Knochendefektstelle 2 angepasst ist, und eine dem Knochendefekt abgewandte Wandung 11 (im Sinne einer Oberfläche bzw. Seite), die der Form des regenerierten Knochens entspricht, auf.

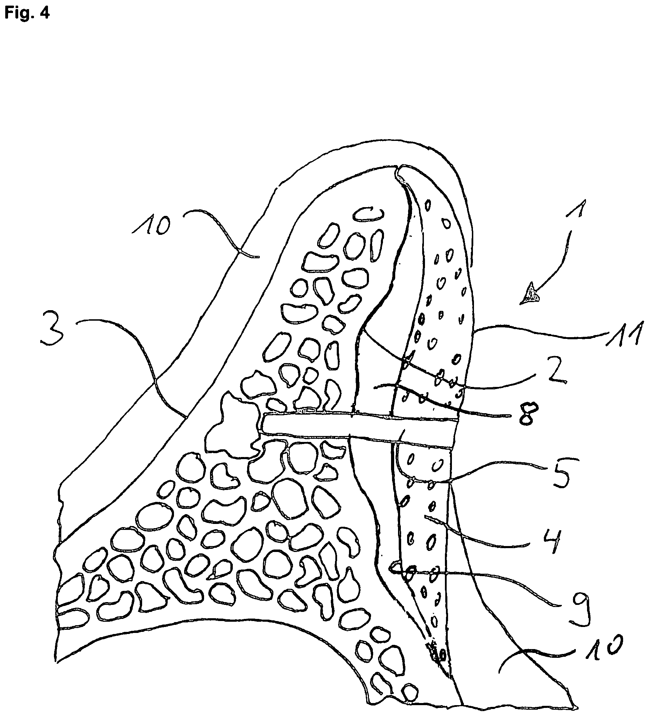

Fig. 4 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist der Aufsatz 4 als Formkörper z.B. aus menschlichen oder tierischen Knochen geformt und weist eine die dem Knochendefekt zugewandte Wandung 9 und eine dem Knochendefekt abgewandte Wandung 11, die der Form des regenerierten Knochens entspricht, auf. Zwischen der Wandung 9 und der Knochendefektstelle 2 befindet sich ein Innenraum 8, der durch die Regeneration des Knochens und/oder durch Einbringung von autogenen, syngenen, allogenen, xenogenen, synthetischen und/oder alloplastischen Material ausgefüllt wird.

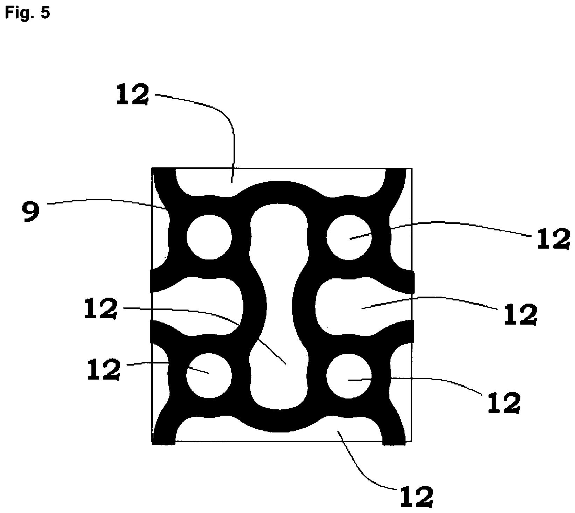

Fig. 5 zeigt einen Ausschnitt eines Aufsatzes 4, dessen dem Knochendefekt zugewandte Wandung 9 Öffnungen 12 aufweist, wodurch eine netzartige Struktur entsteht.

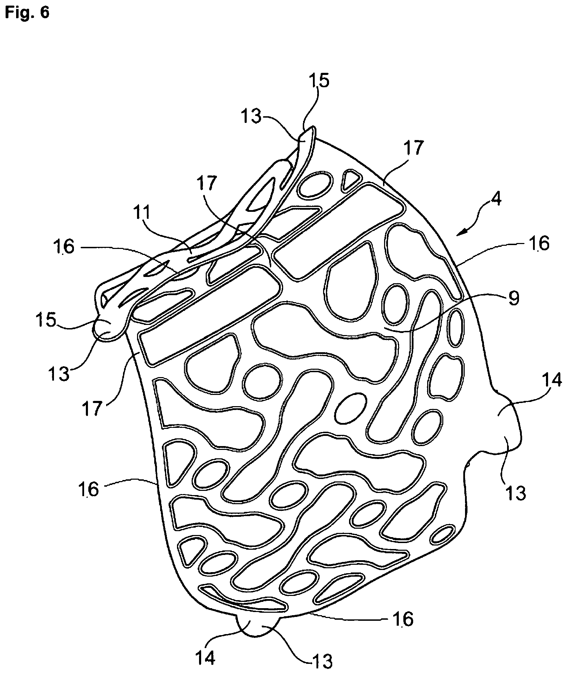

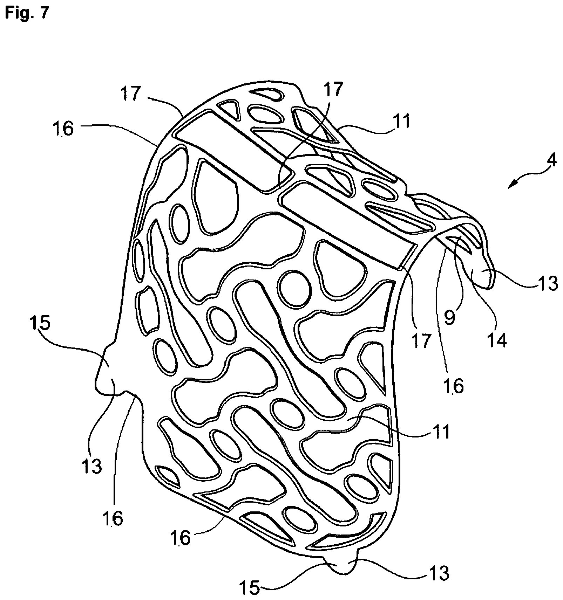

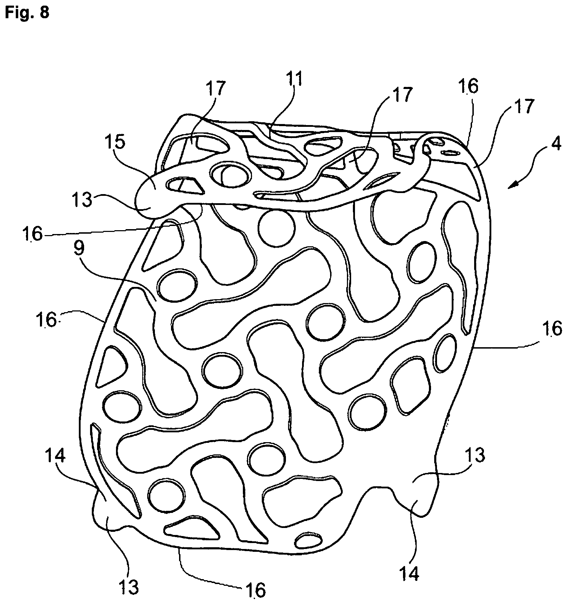

Die Fig. 6 bis 8 zeigen verschiedene Darstellungen eines Aufsatzes 4, der eine einer Knochendefektstelle zugewandte Wandung 9 und eine der Knochendefektstelle abgewandte Wandung 11 aufweist, mit am Rand 16 des Aufsatzes 4 angeordneten öffnungslose Positioniermitteln 13, die eine einem gesunden Knochen zugewandte Wandung 14 und eine dem gesunden Knochen abgewandte Wandung 15 aufweisen. Bei der ordnungsgemäßen Anordnung des Aufsatzes 4 an der Knochendefektstelle, die durch die Positioniermittel 13 unterstützt wird, berühren die einem gesunden Knochen zugewandten Wandungen 14 den gesunden Knochen, wodurch durch die Positioniermittel 13 ein einwandfreier Sitz des Aufsatzes 4 ggfls. selbst ohne Anordnung mindestens eines in den Fig. 6 bis 8 nicht dargestellten Fixiermittels oder zumindest bis mindestens ein Fixiermittel in der Knochendefektstelle angeordnet ist, gewährleistet wird. Um den Aufsatz 4 nach der Knochenregeneration leicht entfernen zu können, weist dieser Sollbruchstellen 17 auf, wodurch er nach deren Durchtrennung für seine Entnahme in zwei Teile geteilt werden kann.

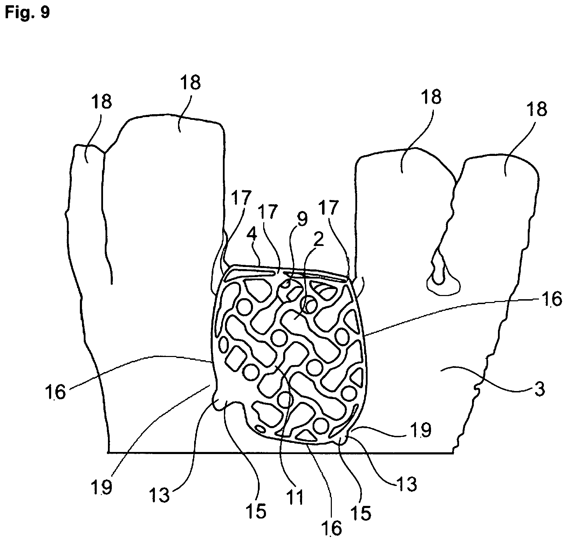

Fig. 9 zeigt einen an einer Knochendefektstelle 2 eines ausschnittsweise dargestellten Kieferknochens 3, der Zähne 18 aufweist, angeordneten Aufsatz 4. Hierdurch wird ersichtlich, dass der Aufsatz 4 bevorzugt nur im Bereich der Knochendefektstelle 2 des Kieferknochens 3 angeordnet ist, so dass er einen gesunden Knochen 19 weder überspannt noch berührt. Somit haben nur die an dem Aufsatz 4 angeordneten Positioniermittel 13 Kontakt mit dem gesunden Knochen 19.

In den Fig. 6 bis 9 ist ein Aufsatz 4 dargestellt, dessen dem Knochendefekt zugewandte Wandung 9 der Form des regenerierten Knochens entspricht. Denkbar ist auch, dass die Positioniermittel 13 derart an dem Aufsatz 4 angeordnet werden, dass dessen dem Knochendefekt abgewandte Wandung 11 der Form des regenerierten Knochens entspricht. Bewerkstelligt werden könnte dies z.B. durch Anordnung der Positioniermittel 13 an der dem Knochendefekt abgewandte Wandung 11 des Aufsatzes 4.

Alle hier dargestellten Merkmale können sowohl einzeln als auch in beliebiger Kombination miteinander erfindungswesentlich sein.

### Bezugszahlenliste

- 1: Vorrichtung
- 2: Knochendefektstelle
- 3: Kieferknochen
- 4: Aufsatz
- 5: Fixiermittel
- 6: Bohrung
- 7: Bohrung
- 8: Innenraum
- 9: Wandung
- 10: Zahnfleisch
- 11: Wandung
- 12: Öffnung
- 13: Positioniermittel
- 14: Wandung
- 15: Wandung
- 16: Rand
- 17: Sollbruchstelle
- 18: Zahn
- 19: Gesunder Knochen

## Patentansprüche

1. Vorrichtung (1) zur Abdeckung und/oder Rekonstruktion einer Knochendefektstelle (2),
- mit einem Aufsatz (4), der einen Rand (16) und eine dem Knochendefekt abgewandte Wandung (11) und eine dem Knochendefekt zugewandte Wandung (9) aufweist,
wobei der Aufsatz (4) aus einem formstabilen Material besteht und eine dem Knochendefekt zugewandte Wandung (9) des Aufsatzes (4) oder eine dem Knochendefekt abgewandte Wandung (11) des Aufsatzes (4) der Form des regenerierten Knochens entspricht,
**dadurch gekennzeichnet,**
**dass** an dem Rand (16) des Aufsatzes (4) mindestens ein öffnungsloses Positioniermittel (13), das eine Wandung (14) aufweist, die einem an den Knochendefekt angrenzenden gesunden Knochen (19) zugewandt ist und mit diesem zumindest teilweise korrespondiert, angeordnet ist
oder
**dass** die Vorrichtung (1) mindestens ein Fixiermittel (5) zur Fixierung des Aufsatzes (4) innerhalb der Knochendefektstelle (2) aufweist und an dem Rand (16) des Aufsatzes (4) mindestens ein öffnungsloses Positioniermittel (13), das eine Wandung (14) aufweist, die einem an den Knochendefekt angrenzenden gesunden Knochen (19) zugewandt ist, und mit diesem zumindest teilweise korrespondiert angeordnet ist.

2. Vorrichtung (1), nach Anspruch 1, **dadurch gekennzeichnet, dass** der Aufsatz (4) und/oder mindestens ein Fixiermittel (5) und/oder mindestens ein Positioniermittel (13) mindestens teilweise aus einem biokompatiblen Werkstoff bestehen.

3. Vorrichtung (1), nach Anspruch 2, **dadurch gekennzeichnet, dass** der Werkstoff des Aufsatzes (4) organischer oder anorganischer Herkunft ist und/oder der Werkstoff eines Fixiermittels (5) organischer oder anorganischer Herkunft ist und/oder der Werkstoff eines Positioniermittel (13) organischer oder anorganischer Herkunft ist.

4. Vorrichtung (1), nach einem vorhergehenden Ansprüche, **dadurch gekennzeichnet, dass** der Aufsatz (4) und/oder mindestens ein Fixiermittel (5) und/oder mindestens ein Positioniermittel (13) mindestens teilweise aus einem biodegradierbaren Werkstoff bestehen.

5. Vorrichtung (1), nach einem vorhergehenden Ansprüche, **dadurch gekennzeichnet, dass** der Aufsatz (4) und/oder mindestens ein Fixiermittel (5) und/oder mindestens ein Positioniermittel (13) mindestens teilweise aus einem resorbierbaren Werkstoff bestehen.

6. Vorrichtung (1), nach einem vorhergehenden Ansprüche, **dadurch gekennzeichnet, dass** der Aufsatz (4) und/oder mindestens ein Fixiermittel (5) und/oder mindestens ein Positioniermittel (13) mindestens teilweise aus einem Polymer oder einer polymeren Verbindung bestehen.

7. Vorrichtung (1), nach einem vorhergehenden Ansprüche, **dadurch gekennzeichnet, dass** der Aufsatz (4) und/oder mindestens ein Fixiermittel (5) und/oder mindestens ein Positioniermittel (13) mindestens teilweise aus Polylactid bestehen.

8. Vorrichtung (1), nach einem vorhergehenden Ansprüche, **dadurch gekennzeichnet, dass** der Aufsatz (4) eine variierende Wandstärke aufweist und/oder mindestens ein Positioniermittel (13) eine variierende Wandstärke aufweist.

9. Vorrichtung (1), nach Anspruch 8, **dadurch gekennzeichnet, dass** die Wandstärke mindestens 0,2 mm beträgt.

10. Vorrichtung (1), nach einem vorhergehenden Ansprüche, **dadurch gekennzeichnet, dass** das Fixiermittel (5) ein Pin, eine Schraube, ein Nagel und/oder ein Knochenkleber ist.

11. Vorrichtung (1), nach einem vorhergehenden Ansprüche, **dadurch gekennzeichnet, dass** der Aufsatz (4) mindestens eine Fräsung aufweist.

12. Vorrichtung (1), nach Anspruch 11, **dadurch gekennzeichnet, dass** die Fräsung mit dem Fixiermittel (5) korrespondiert.

13. Vorrichtung (1), nach einem vorhergehenden Ansprüche, **dadurch gekennzeichnet, dass** die dem Knochendefekt zugewandte Wandung (9) eine Oberflächenkonditionierung aufweist.

14. Vorrichtung (1), nach Anspruch 13, **dadurch gekennzeichnet, dass** die Oberflächenkonditionierung eine Mikrostrukturierung, Poren, Osteoblastenlockstoffe, Mittel zur Förderung des Knochenwachstums und/oder BMP-haltiges Knochenersatzmittel aufweist.

15. Vorrichtung (1), nach einem vorhergehenden Ansprüche, **dadurch gekennzeichnet, dass** der Aufsatz (4) mindestens eine Öffnung aufweist.

16. Vorrichtung (1), nach einem vorhergehenden Ansprüche, **dadurch gekennzeichnet, dass** der Aufsatz (4) mindestens eine Sollbruchstelle (16) aufweist.

17. Vorrichtung (1), nach einem vorhergehenden Ansprüche **dadurch gekennzeichnet, dass** der Aufsatz (4) mindestens eine Befestigungsvorrichtung für mindestens ein zu setzendes Implantat aufweist.

18. Vorrichtung (1), nach Anspruch 17, **dadurch gekennzeichnet, dass** mindestens eine Befestigungsvorrichtung durch einen Teil des Aufsatzes (4), der mittels mindestens einer Sollbruchstelle (16) mit dem restlichen Teil des Aufsatzes (4) verbunden ist, zumindest teilweise abgedeckt ist.

19. Vorrichtung (1), nach einem vorhergehenden Ansprüche, **dadurch gekennzeichnet, dass** zwischen dem Aufsatz (4) und einem Positioniermittel (13) mindestens eine Sollbruchstelle (16) angeordnet ist.

20. Verfahren zur Herstellung eines Aufsatzes (4) einer Abdeckvorrichtung für eine Knochendefektstelle (2), bestehend aus folgenden Verfahrensschritten:
- Aufnahme eines ersten Datensatzes, der die betroffene Knochendefektstelle (2) im Ist-Zustand repräsentiert,
- Vergleich des ersten Datensatzes mit einem zweiten Datensatz, der den Soll-Zustand eines an der Knochendefektstelle (2) regenerierten Knochens repräsentiert, wobei der zweite Datensatz durch Berechnung entsteht oder zu einer Zeit aufgenommen wurde, zu der der Knochen an der jetzt zu regenerierenden Stelle noch ein gesunder Knochen (19) war,
- Verwendung des ersten Datensatzes und des zweiten Datensatzes zur Planung des Aufsatzes (4), der einen Rand (16) und eine dem Knochendefekt abgewandte Wandung (11) und eine dem Knochendefekt zugewandte Wandung (9) aufweist, wodurch der Aufsatz (4) ausschließlich im Bereich der Knochendefektstelle anordbar ist, und ggfls. mit mindestens einem Fixiermittel (5) an einem Knochen fixierbar ist,
- Umsetzung der Planung des Aufsatzes (4) in einen Planungsdatensatz und
- Zuführung des Planungsdatensatzes an ein Fertigungsverfahren, insbesondere an ein computergesteuertes Fertigungsverfahren, in dem der Aufsatz (4) aus einem formstabilen Material gebildet wird und dessen dem Knochendefekt zugewandte Wandung (9) oder dessen dem Knochendefekt abgewandte Wandung (11) der Form des regenerierten Knochens im Soll-Zustand entspricht, wobei während der Fertigung des Aufsatzes (4) an dem Rand (16) des Aufsatzes (4) mindestens ein Positioniermittel (13) angeordnet wird, das zur Positionierung des Aufsatzes (4) an einem an die Knochendefektstelle (2) angrenzenden gesunden Knochen (19) dient, wobei ein öffnungsloses Positioniermittel (13) eine dem gesunden Knochen (19) abgewandte Wandung (15) und eine dem gesunden Knochen (19) und mit diesem zumindest teilweise korrespondierende zugewandte Wandung (14) aufweist.

21. Verfahren, nach Anspruch 20, **dadurch gekennzeichnet, dass** die Aufnahme des ersten Datensatzes die betroffene Knochendefektstelle (2) in ihrer Dreidimensionalität repräsentiert und/oder die Aufnahme des zweiten Datensatzes die Form des noch gesunden Knochens (19) in seiner Dreidimensionalität repräsentiert.

22. Verfahren, nach Anspruch 20 oder Anspruch 21, **dadurch gekennzeichnet, dass** die Aufnahme des ersten Datensatzes und/oder die Aufnahme des zweiten Datensatzes durch mindestens ein bildgebendes Verfahren erfolgt.

23. Verfahren, nach einem der Ansprüche 20 bis 22, **dadurch gekennzeichnet, dass** die Aufnahme des ersten Datensatzes und/oder die Aufnahme des zweiten Datensatzes mit mindestens einem Verfahren, welches eine dreidimensionale Darstellung eines Knochens ermöglicht, erfolgen.

24. Verfahren zur Herstellung eines Aufsatzes (4), nach einem der Ansprüche 20 bis 23, **dadurch gekennzeichnet, dass** die Aufnahme des Datensatzes des gesunden Knochens (19) erfolgt, nachdem der gesunde Knochen (19) ausgewachsen ist.

25. Verfahren zur Herstellung eines Aufsatzes (4), nach Anspruch 24, **dadurch gekennzeichnet, dass** der Datensatz des gesunden Knochens (19) für seine spätere Verwendung auf einem Speichermedium gespeichert wird.

26. Verfahren zur Herstellung eines Aufsatzes (4), nach einem der Ansprüche 20 bis 25, **dadurch gekennzeichnet, dass** im Fertigungsverfahren der Aufsatz (4) mittels Fräsung gebildet wird.

27. Verfahren zur Herstellung eines Aufsatzes (4), nach einem der Ansprüche 20 bis 26, **dadurch gekennzeichnet, dass** während der Fertigung des Aufsatzes (4) mindestens eine Befestigungsvorrichtung für mindestens ein zu setzendes Implantat an dem Aufsatz (4) angeordnet wird.

28. Verfahren zur Herstellung eines Aufsatzes (4), nach Anspruch 27, **dadurch gekennzeichnet, dass** mindestens eine Befestigungsvorrichtung durch Entfernen eines Teils des Aufsatzes (4), der vor dem Entfernen mittels mindestens einer Sollbruchstelle (16) mit dem restlichen Teil des Aufsatzes (4) verbunden ist, freigelegt wird.

29. Verfahren zur Herstellung eines Aufsatzes (4), nach einem der Ansprüche 20 bis 28, **dadurch gekennzeichnet, dass** während der Fertigung des Aufsatzes (4) mindestens eine Sollbruchstelle (16) an dem Aufsatz (4) angeordnet wird.

30. Verfahren zur Herstellung eines Aufsatzes (4), nach einem der Ansprüche 20 bis 29, **dadurch gekennzeichnet, dass** zwischen dem Aufsatz (4) und einem Positioniermittel (13) mindestens eine Sollbruchstelle (16) angeordnet wird.

31. Verfahren zur Herstellung eines Aufsatzes (4), nach einem der Ansprüche 20 bis 30, **dadurch gekennzeichnet, dass** während der Fertigung des Aufsatzes (4) ein Reinigungs- und/oder Sterilisationsprozess durchgeführt wird.

32. Verfahren zur Herstellung eines Aufsatzes (4), nach einem der Ansprüche 20 bis 31, **dadurch gekennzeichnet, dass** der Aufsatz in einer Vorrichtung zur Abdeckung und/oder Rekonstruktion einer Knochendefektstelle gemäß den Ansprüchen 1 bis 19 einsetzbar ist.

## Claims

1. A device (1) for covering and/or reconstructing a bone defect site (2),
- having a cap (4) which has an edge (16) and a wall (11) directed away from the bone defect and a wall (9) directed towards the bone defect,
wherein the cap (4) is made of a dimensionally stable material and a wall (9) of the cap (4) directed towards the bone defect or a wall (11) of the cap (4) directed away from the bone defect corresponds to the shape of the regenerated bone, **characterized in that**
at least one positioning means (13) without an opening is arranged on the edge (16) of the cap (4), said positioning means having a wall (14) directed towards a healthy bone (19) adjacent to the bone defect and corresponding thereto, at least in part,
or
that the device (1) has at least one fixing means (5) for fixing the cap (4) within the bone defect site (2) and at least one positioning means (13) without an opening on the edge (16) of the cap (4), said positioning means having a wall (14) directed towards a healthy bone (19) adjacent to the bone defect and corresponding thereto, at least in part.

2. The device (1) according to claim 1, **characterized in that** the cap (4) and/or at least one fixing means (5) and/or at least one positioning means (13) are composed of a biocompatible material, at least in part.

3. The device (1) according to claim 2, **characterized in that** the material of the cap (4) is of organic or inorganic origin and/or the material of a fixing means (5) is of organic or inorganic origin and/or the material of a positioning means (13) is of organic or inorganic origin.

4. The device (1) according to one of the preceding claims, **characterized in that** the cap (4) and/or at least one fixing means (5) and/or at least one positioning means (13) are composed of a biodegradable material, at least in part.

5. The device (1) according to one of the preceding claims, **characterized in that** the cap (4) and/or at least one fixing means (5) and/or at least one positioning means (13) are composed of an absorbable material, at least in part.

6. The device (1) according to one of the preceding claims, **characterized in that** the cap (4) and/or at least one fixing means (5) and/or at least one positioning means (13) are composed of a polymer or a polymer compound, at least in part.

7. The device (1) according to one of the preceding claims, **characterized in that** the cap (4) and/or at least one fixing means (5) and/or at least one positioning means (13) are composed of polylactide, at least in part.

8. The device (1) according to one of the preceding claims, **characterized in that** the cap (4) has a varying wall thickness and/or at least one positioning means (13) has a varying wall thickness.

9. The device (1) according to claim 8, **characterized in that** the wall thickness is at least 0.2 mm.

10. The device (1) according to one of the preceding claims, **characterized in that** the fixing means (5) is a pin, a screw, a nail and/or a bone adhesive.

11. The device (1) according to one of the preceding claims, **characterized in that** the cap (4) has at least one milled region.

12. The device (1) according to claim 11, **characterized in that** milled region corresponds to the fixing means (5).

13. The device (1) according to one of the preceding claims, **characterized in that** the wall (9) directed towards the bone defect exhibits surface conditioning.

14. The device (1) according to claim 13, **characterized in that** the surface conditioning has a microstructure, pores, osteoblast attraction substances, bone growth promoting agents and/or bone replacement material that contains a bone morphogenetic protein.

15. The device (1) according to one of the preceding claims, **characterized in that** the cap (4) has at least one opening.

16. The device (1) according to one of the preceding claims, **characterized in that** the cap (4) has at least one predetermined breaking point (16).

17. The device (1) according to one of the preceding claims, **characterized in that** the cap (4) has at least one fixing device for at least one implant which is to be fitted.

18. The device (1) according to claim 17, **characterized in that** at least one fixing device is covered, at least in part, by a part of the cap (4) which is connected by means of at least one predetermined breaking point (16) to the remaining part of the cap (4).

19. The device (1) according to one of the preceding claims, **characterized in that** at least one predetermined breaking point (16) is arranged between the cap (4) and a positioning means (13).

20. A method for producing a cap (4) of a covering device for a bone defect site (2) comprising the following method steps:
- recording of a first data set which represents the affected bone defect site (2) in its actual state,
- comparison of the first data set with a second data set which represents the target state of a regenerated bone at the bone defect site (2), wherein the second data set is produced by computation or is recorded at a time when the bone at the site now being regenerated was still a healthy bone (19),
- use of the first data set and the second data set for planning the cap (4) which has an edge (16) and a wall (11) directed away from the bone defect and a wall (9) directed towards the bone defect, wherein the cap (4) can be exclusively arranged in the region of the bone defect site and can be fixed to a bone where necessary using at least one fixing means (5),
- implementation of the planning of the cap (4) as a planning data set and
- provision of the planning data set to a production method, in particular to a computer-controlled production method, in which the cap (4) is created from a dimensionally stable material and whereof the wall (9) directed towards the bone defect or whereof the wall (11) directed away from the bone defect corresponds to the shape of the regenerated bone in the target state, wherein during the production of the cap (4), at least one positioning means (13) is arranged on the edge (16) of the cap (4), which positioning means is used for positioning the cap (4) on a healthy bone (19) adjacent to the bone defect site (2), wherein a positioning means (13) without an opening has a wall (15) directed away from the healthy bone (19) and a wall (14) directed towards the healthy bone (19) and corresponding thereto, at least in part.

21. The method according to claim 20, **characterized in that** the recording of the first data set represents the affected bone defect site (2) in its three-dimensionality and/or the recording of the second data set represents the shape of the bone (19) which is still healthy in its three-dimensionality.

22. The method according to claim 20 or claim 21, **characterized in that** the recording of the first data set and/or the recording of the second data set takes place using at least one imaging method.

23. The method according to one of claims 20 to 22, **characterized in that** the recording of the first data set and/or the recording of the second data set takes place using at least one method which allows a three-dimensional representation of a bone.

24. The method for producing a cap (4) according to one of claims 20 to 23, **characterized in that** the recording of the data set of the healthy bone (19) takes place once the healthy bone (19) is fully grown.

25. The method for producing a cap (4) according to claim 24, **characterized in that** the data set of the healthy bone (19) is stored for subsequent use on a storage medium.

26. The method for producing a cap (4) according to one of claims 20 to 25, **characterized in that** the cap (4) is formed in the production method by means of milling.

27. The method for producing a cap (4) according to one of claims 20 to 26, **characterized in that** during production of the cap (4), at least one fixing device for at least one implant to be fitted is arranged on the cap (4).

28. The method for producing a cap (4) according to claim 27, **characterized in that** at least one fixing device is exposed by removing part of the cap (4) which is connected to the remaining part of the cap (4) prior to removal by means of at least one predetermined breaking point (16).

29. The method for producing a cap (4) according to one of claims 20 to 28, **characterized in that** during production of the cap (4) at least one predetermined breaking point (16) is arranged on the cap (4).

30. The method for producing a cap (4) according to one of claims 20 to 29, **characterized in that** at least one predetermined breaking point (16) is arranged between the cap (4) and a positioning means (13).

31. The method for producing a cap (4) according to one of claims 20 to 30, **characterized in that** a cleaning and/or sterilization process is carried out during production of the cap (4).

32. The method for producing a cap (4) according to one of claims 20 to 31, **characterized in that** the cap can be used in a device for covering and/or reconstructing a bone defect site according to claims 1 to 19.

## Revendications

1. Dispositif (1) de recouvrement et/ou de reconstruction d'un point osseux défectueux (2),
- comportant une corniche (4) qui présente un bord (16) et une paroi (11) détournée du point osseux défectueux et une paroi (9) tournée vers le point osseux défectueux,
la corniche (4) étant composée d'un matériau à forme stable et une paroi (9) tournée vers le point osseux défectueux de la corniche (4) ou une paroi (11) détournée du point osseux défectueux de la corniche (4) correspondant à la forme de l'os régénéré, **caractérisé en ce que**,
au bord (16) de la corniche (4), au moins un moyen de positionnement sans ouverture (13), qui présente une paroi (14) qui est tournée vers l'os sain (19) adjacent au défaut osseux et correspond partiellement à celui-ci, est disposé ou
que le dispositif (1) présente au moins un moyen de fixation (5) pour la fixation de la corniche (4) à l'intérieur du point osseux défectueux (2) et qu'est disposé au bord (16) de la corniche (4) au moins un moyen de positionnement sans ouverture (13) qui présente une paroi (14) qui est tournée vers un os sain (19) adjacent au défaut osseux et lui correspond du moins partiellement.

2. Dispositif (1) selon la revendication 1, **caractérisé en ce que** la corniche (4) et/ou au moins un moyen de fixation (5) et/ou au moins un moyen de positionnement (13) sont composés du moins partiellement d'un matériau biocompatible.

3. Dispositif (1) selon la revendication 2, **caractérisé en ce que** le matériau de la corniche (4) est d'origine organique ou anorganique et/ou le matériau d'un moyen de fixation (5) est d'origine organique ou anorganique et/ou le matériau d'un moyen positionnement (13) est d'origine organique ou anorganique.

4. Dispositif (1) selon une des revendications précédentes, **caractérisé en ce que** la corniche (4) et/ou au moins un moyen de fixation (5) et/ou au moins un moyen de positionnement (13) sont composés du moins partiellement d'un matériau biodégradable.

5. Dispositif (1) selon une des revendications précédentes, **caractérisé en ce que** la corniche (4) et/ou au moins un moyen de fixation (5) et/ou au moins un moyen de positionnement (13) sont composés du moins partiellement d'un matériau résorbable.

6. Dispositif (1) selon une des revendications précédentes, **caractérisé en ce que** la corniche (4) et/ou au moins un moyen de fixation (5) et/ou au moins un moyen de positionnement (13) sont composés du moins partiellement d'un composé polymère.

7. Dispositif (1) selon une des revendications précédentes, **caractérisé en ce que** la corniche (4) et/ou au moins un moyen de fixation (5) et/ou au moins un moyen de positionnement (13) sont composés du moins partiellement de polylactide.

8. Dispositif (1) selon une des revendications précédentes, **caractérisé en ce que** la corniche (4) présente une épaisseur de paroi variable et/ou qu'au moins un moyen de positionnement (13) présente une épaisseur de paroi variable.

9. Dispositif (1) selon la revendication 8, **caractérisé en ce que** l'épaisseur de paroi s'élève à au moins 0,2 mm.

10. Dispositif (1) selon une des revendications précédentes, **caractérisé en ce que** le moyen de fixation (5) est une broche, une vis, un clou et/ou une colle à os.

11. Dispositif (1) selon une des revendications précédentes, **caractérisé en ce que** la corniche (4) présente au moins un fraisage.

12. Dispositif (1) selon la revendication 11, **caractérisé en ce que** le fraisage correspond au moyen de fixation (5).

13. Dispositif (1) selon une des revendications précédentes, **caractérisé en ce que** la paroi (9) tournée vers le défaut osseux présente un conditionnement superficiel.

14. Dispositif (1) selon la revendication 13, **caractérisé en ce que** le conditionnement superficiel présente une micro-structuration, des pores, des substances attractives d'ostéoblastes, des moyens de stimulation de la croissance osseuse et/ou des substituts osseux contenant des BMP.

15. Dispositif (1) selon une des revendications précédentes, **caractérisé en ce que** la corniche (4) présente au moins une ouverture.

16. Dispositif (1) selon une des revendications précédentes, **caractérisé en ce que** la corniche (4) présente au moins un point de rupture programmée (16).

17. Dispositif (1) selon une des revendications précédentes, **caractérisé en ce que** la corniche (4) présente au moins un dispositif de fixation pour au moins un implant à poser.

18. Dispositif (1) selon la revendication 17, **caractérisé en ce qu'**au moins un dispositif de fixation est recouvert du moins partiellement par une partie de la corniche (4) qui est connectée au moyen d'au moins un point de rupture programmée (16) à la partie restante de la corniche (4).

19. Dispositif (1) selon une des revendications précédentes, **caractérisé en ce que**, entre la corniche (4) et un moyen de positionnement (13), au moins un point de rupture programmée (16) est disposé.

20. Procédé de réalisation d'une corniche (4) d'un dispositif de recouvrement pour un point osseux défectueux (2), composé des étapes opératoires suivantes :
- enregistrement d'un premier jeu de données qui représente le point osseux défectueux concerné (2) en état réel,
- comparaison du premier jeu de données à un second jeu de données qui représente l'état théorique d'un os régénéré au niveau du point osseux défectueux (2), le second jeu de données étant créé par calcul ou ayant été enregistré à un moment où l'os était encore un os sain (19) au point à régénérer maintenant,
- utilisation du premier jeu de données et du second jeu de données pour concevoir la corniche (4) qui présente un bord (16) et une paroi (11) détournée du défaut osseux et une paroi (9) tournée vers le défaut osseux, ce qui a pour effet que la corniche (4) peut être disposée exclusivement au niveau du point osseux défectueux et peut être fixée le cas échéant à un os au moyen d'au moins un moyen de fixation (5),
- transposition de la conception de la corniche (4) dans un jeu de données de conception et
- acheminement du jeu de données de conception vers un procédé de fabrication, en particulier un procédé de fabrication contrôlé par ordinateur, dans lequel la corniche (4) est constituée d'un matériau de forme stable et dont la paroi (9) tournée vers le défaut osseux ou la paroi (11) détournée du défaut osseux correspond à la forme de l'os régénéré en état réel, sachant que, pendant la fabrication de la corniche (4), au bord (16) de la corniche (4), est disposé au moins un moyen de positionnement (13) qui sert à positionner la corniche (4) au niveau d'un os sain (19) adjacent au point osseux défectueux, un moyen de positionnement sans ouverture (13) présentant une paroi (15) détournée de l'os sain (19) et une paroi (14) lui correspondant au moins partiellement tournée vers l'os sain (19).

21. Procédé selon la revendication 20, **caractérisé en ce que** l'enregistrement du premier jeu de données représente le point osseux défectueux concerné (2) dans sa tridimensionnalité et/ou l'enregistrement du second jeu de données représente la forme de l'os encore sain (19) dans sa tridimensionnalité.

22. Procédé selon la revendication 20 ou la revendication 21, **caractérisé en ce que** l'enregistrement du premier jeu de données et/ou l'enregistrement du second jeu de données se fait par au moins un procédé d'imagerie.

23. Procédé selon une des revendications 20 à 22, **caractérisé en ce que** l'enregistrement du premier jeu de données et/ou l'enregistrement du second jeu de données se fait par au moins un procédé qui permet une représentation tridimensionnelle d'un os.

24. Procédé de fabrication d'une corniche (4) selon une des revendications 20 à 23, **caractérisé en ce que** l'enregistrement du jeu de données de l'os sain (19) se fait une fois que l'os sain (19) a poussé.

25. Procédé de fabrication d'une corniche (4) selon la revendication 24, **caractérisé en ce que** le jeu de données de l'os sain (19) est sauvegardé sur un support de mémoire pour son utilisation ultérieure.

26. Procédé de fabrication d'une corniche (4) selon une des revendications 20 à 25, **caractérisé en ce que** la corniche (4) est formée par fraisage dans le procédé de fabrication.

27. Procédé de fabrication d'une corniche (4) selon une des revendications 20 à 26, **caractérisé en ce que**, pendant la fabrication de la corniche (4), au moins un dispositif de fixation pour au moins un implant à poser est disposé sur la corniche (4).

28. Procédé de fabrication d'une corniche (4) selon la revendication 27, **caractérisé en ce qu'**au moins un moyen de fixation est dégagé en enlevant une partie de la corniche (4) qui, avant son enlèvement, est connectée au moyen d'un point de rupture programmée (16) à la partie restante de la corniche (4).

29. Procédé de fabrication d'une corniche (4) selon une des revendications 20 à 28, **caractérisé en ce que**, pendant la fabrication de la corniche (4), au moins un point de rupture programmée (16) est disposé au niveau de la corniche (4).

30. Procédé de fabrication d'une corniche (4) selon une des revendications 20 à 29, **caractérisé en ce que**, entre la corniche (4) et au moins un moyen de positionnement (13), au moins un point de rupture programmée (16) est disposé.

31. Procédé de fabrication d'une corniche (4) selon une des revendications 20 à 30, **caractérisé en ce que**, pendant la fabrication de la corniche (4), un processus de nettoyage et/ou de stérilisation est exécuté.

32. Procédé de fabrication d'une corniche (4) selon une des revendications 20 à 31, **caractérisé en ce que** la corniche est insérable dans un dispositif de recouvrement et/ou de reconstruction d'un point osseux défectueux selon les revendications 1 à 19.
